# EUROPÄISCHE PATENTSCHRIFT

(11) **EP 0 537 547 B1**
(45) Veröffentlichungstag und Bekanntmachung des Hinweises auf die Patenterteilung: **22.01.1997**
(21) Anmeldenummer: 92116641.9
(22) Anmeldetag: 29.09.1992
(51) Int. Cl.: C12F 3/06, A21D 8/04, C12N 1/18

(54) **Der Gebrauch von Bierhefe für die industrielle Erzeugung der Bäckerhefe**
Use of brewers yeast for the industrial production of bakers yeast
Utilisation de levure de bière pour la production industrielle de levure de boulangerie

(30) Priorität: 30.09.1991 YU 1596/91
(43) Veröffentlichungstag der Anmeldung: 21.04.1993
(73) Patentinhaber: Milosavljevic, Aleksandar, YU-35000 Svetozarevo (YU)
(72) Erfinder: Milosavljevic, Aleksandar, YU-35000 Svetozarevo (YU)
(74) Vertreter: Patentanwälte Zellentin & Partner

(56) Entgegenhaltungen:
- GB-A- 106 531
- GB-A- 117 666
- GB-A- 149 533

## Beschreibung

Diese Erfindung bezieht sich auf die Bierhefe- und die Bäckerhefetechnologie.

### Technisches Problem

Die neuen Verfahren ermöglichen die Verbindung zweier bis jetzt unabhängiger und physisch getrennter technologischer Verfahren mit ähnlichen biochemischen Prozessen. In erster Linie ermöglichen die Verfahren die Vereinigung der technologischen Verfahren der Biererzeugung und der Bäckerhefeerzeugung in einem Verfahren.

### Stand der Technik

Bierhefe entsteht beim Verfahren der Biererzeugung als Nebenprodukt. Sie wurde bis jetzt als Viehfutter gebraucht, nachdem sie mit anderen Produkten getrocknet wurde. Manche Brauereien verkaufen Bierhefe nach der Entbitterung durch Waschen mit 1% Lösung NaOH und Trocknung als Additiv. Manche Fabriken werfen die Bierhefe weg und verschmutzen auf diese Weise die Umwelt.

### Technisches Problem

Auf die Idee, Bierhefe für die industrielle Erzeugung von Bäckerhefe zu gebrauchen, bin ich gekommen, indem ich von der Tatsache ausging, daß es sich um die gleiche Art Hefe SACCHAROMYCES CEREVISIAE handelt und daß die Hefe, die die alkoholische Fermentation bei der Biererzeugung verrichtet, das auch besser und schneller tun könne oder genauso gut wie die Bäckerhefe, da man annehmen könne, daß die Hefe schon auf diesen Prozess adaptiert ist. In der Praxis war es ganz anders. Nach der beendeten Fermentation und Sedimentation im Lagertank erhält man die Bierhefe zusammen mit anderen Teilchen der Sedimentation aus der Würze und aus dem Hopfenharz. Das Hopfenharz gibt der Hefe einen bitteren Geschmack, Klebrigkeit und eine dunkle Farbe, was die weitere Anwendung in der Industrie der Bäckerhefe begrenzt. Ein anderer und entscheidender Faktor für die Nichtanwendung in der Bäckerindustrie war die schlechte Teighebung. Da ich mehrere Proben auf Teighebung gemacht habe, habe ich festgestellt, daß die Teighebung zwischen 160 bis 170 Minuten und mehr dauert, was drei bis viermal länger als bei Bäckerhefe ist. Um dies zu überprüfen, habe ich folgendes Experiment gemacht:
- Zuerst habe ich die Entbitterung der Bierhefe mit 1% Lösung von Na₂ CO₃ vorgenommen, die Hefe im Verhältnis 1:5 gemischt, und dann mehrmals mit Wasser gewaschen (dieses Verfahren ist bei der Verwendung von Bierhefe zur Erzeugung von Additiven bekannt, s. die Zeitschrift Brauerein aus dem Jahre 1972 - viertes Symposium).

Das alles habe ich in einen Trenntrichter gegeben, dann vakuumfiltriert und so eine erhöhte Konzentration an reiner entbitterter und gewaschener Bierhefe in der Hefemilch bekommen. Die abfiltrierte Hefe habe ich zur Teighebung verwendet und dann Brot gebacken. Den Rest der Hefe habe ich zur Prüfung auf Dauerhaftigkeit stehen gelassen. Ich habe folgende Resultate bekommen:

| Probe aus dem Lagertank | S.M% | Feuchte | Protein% | Zeit der Teighebung | | | | % der toten Zellen |
|---|---|---|---|---|---|---|---|---|
| | | | | I | II | III | Summe | |
| Bierhefe unentbittert | 25,2 | 74,8 | 49,35 | 103' | 33' | 29' | 165 | 5 |
| entbittert mit 1%Na₂Co₃ | 25,1 | 74,9 | 49,35 | 160' | 50' | 25' | 235' | 40 |
| entbittert mit 0,1 % Na₂ CO₃ | 25,2 | 74,8 | 49,35 | 102' | 33' | 28' | 163' | 5 |

| Probe 2. | S.M% | Feuchte | Protein% | Zeit der Teighebung | | | | der toten Zellen % |
|---|---|---|---|---|---|---|---|---|
| | | | | I | II | III | Summe | |
| Bierhefe unentbittert | 25,5 | 74,5 | 50,07 | 109' | 35' | 28' | 172' | 10 |
| Entbittert mit 1% Na₂CO₃ | 25,6 | 74,4 | 50,07 | 195' | 60' | 30' | 285' | 50 |
| Entbittert mit 0,1 % Na₂CO₃ | 25,6 | 74,4 | 50,07 | 111' | 31' | 25' | 167' | 10 |

SCHLUSSFOLGERUNG: Bei mehrere Male wiederholten Untersuchungen gab es keine bedeutenden Unterschiede. Bei allen Experimenten habe ich eine mehr oder weniger sehr hohe Zeit der Teighebung festgestellt, die sich mit dem Prozent der toten Zellen vermehrte (Färbung mit Methylblau). Ich habe auch festgestellt, daß sich der Prozentsatz der toten Zellen mit der Vergrößerung der Konzentration an Na₂CO₃ zur Entbitterung der Bierhefe vergrößerte. Mit dem Mikroskop wurde eine starke Agglutination der Hefe festgestellt, es wurden große Flocken gebildet, die sich sehr schnell ablagern und sich ganz von der flüßigen Phase trennen.

Weiter habe ich Experimente mit Bierhefe gemacht, indem ich Bierhefe mit 0,1% Lösung Na₂CO₃ entbittert und mit Wasser 5 bis 6 mal im Verhältnis 1:5 gewaschen und dann mit Hefemilch gemischt habe, und zwar mit Hefemilch aus dem Prozess der Erzeugung von Bierhefe im Verhältnis von: 10%, 30% und 50%. Ich habe folgende Resultate bekommen:

| | S.M.% | N/N s.m.% | Protein | Feuchte % | Zeit der Teighebung | | | | Dauerhaftigkeit bei 35°C |
|---|---|---|---|---|---|---|---|---|---|
| | | | | | I | II | III | Summe | |
| Bierhefe | 25,1 | 2,01/8,1 | 50,07 | 74,9 | 102' | 33' | 28' | 163 | 168 Std. |
| Bäckerhefe | 25,3 | 1,7/6,92 | 43,25 | 74,7 | 43' | 8' | 8' | 59' | 168 Std. |
| Mischung von 10% | 25,2 | 1,79/7,15 | 44,69 | 74,8 | 42' | 8' | 7' | 57' | 168 Std. |
| Mischung von 30% | 25,0 | 1,85/7,4 | 46,25 | 75,0 | 42' | 9' | 7' | 58' | 168 Std. |
| Mischung von 50% | 25,2 | 1,99/7,9 | 49,38 | 74,8 | 43' | 9' | 7' | 59' | 168 Std. |

In den mehrmals wiederholten Experimenten habe ich fast identische Resultate bekommen, so daß man die Resultate der obigen Tabelle als durchschnittlich ansehen kann. Aus den erhaltenen Ergebnissen kann man folgenden Schluß ziehen: Wenn man Hefemilch aus entbitterter und gewaschener Bierhefe, die den Teig in 163 Minuten hebt, in den Verhältnissen 10%, 30% und 50% mit Hefemilch aus Bäckerhefe mischt, bekommt man eine Teighebung, die der Hebung aus reiner Hefemilch gleich ist. Es ist auch die Dauerhaftigkeit bei 35°C gleich.

Bei der Geschmacksprüfung dieses Brotes bemerkte man keinen Unterschied in der Qualität aber am Geschmack und Geruch eine bedeutende Verbesserung. Ich habe somit bestätigt, daß gewaschene Bierhefe ohne Problem als Bäckerhefe gebraucht werden kann, und man somit die Qualität der Bäckerhefe verbessert.

Das vorgeschlagene Verfahren zur Entbitterung und zum Waschen ist ein sehr rationelles und gründet sich auf die ausgezeichnete Fähigkeit der Bierhefe abzulagern und der leichten Dekantierung der Phasen.

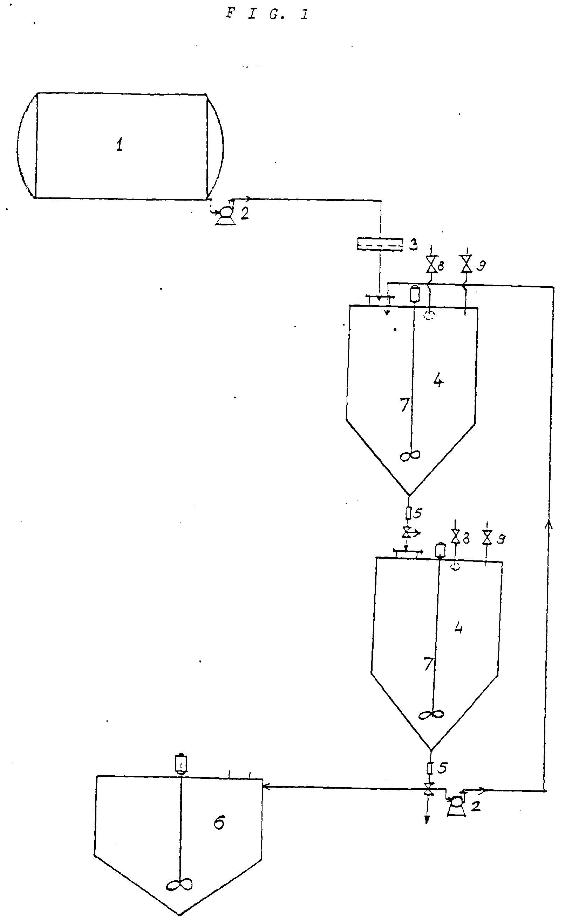

Erläuterung der Vorrichtung und des Verfahrens an Hand des Schemas gemäß Fig. 1

Bierhefe wird aus dem Fermentor 1 nach beendeter Fermentation mittels einer Pumpe 2 auf ein Vibrationssieb 3 übergeben, wo sich die Teilchen der Sedimentation aus Hopfenharz und Protein abtrennen. Die gereinigte Hefe wird in einen ersten Bottich 4 mit einem Rührwerk 7 zugeführt, wo die Entbitterung unter Rühren mit 0,1% Na₂CO₃-Lösung oder 0,1% NaHCO₃-Lösung im Verhältnis 1:3 bis 1:5 durchgeführt wird. Dann läßt man fünf Minuten stehen und dekantiert so, daß die erste Phase mit der groben Sedimentation aus Hopfenharz abgetrennt wird, was mittels einer Sichtlaterne 5 beobachtbar ist. Die zweite Phase pumpt man in einen zweiten Bottich 4 und die dritte Phase wird verworfen. Die in den zweiten Bottich 4 mit Rührwerk 7 gepumpte Hefe wird mit Wasser im gleichen Verhältnis 1:3 bis 1:5 vermischt gewaschen und dann wird fünf Minuten gerührt, danach zehn Minuten stehengelassen und es wird wieder dekantiert. Die gewaschene Hefe wird mittels der Pumpe 2 in den ersten Bottich 4 zum Waschen gepumpt und das gleiche Verfahren wird 3 bis 5 mal wiederholt. Die so entbitterte und gewaschene Hefe wird in einen dritten Bottich 6 gepumpt, wo sie mit Hefemilch aus der separierten Bäckerhefe in der Quantität bis 50% gut verrührt wird. Die Mischung wird dann im Vakuumfilter filtriert und für den Markt verpackt.

Das Verfahren des Waschens und der Entbitterung wurde an verschiedenen Arten von Hefen Saccharomyces untersucht. Es wurde dabei festgestellt, daß obergärigen Hefen, sogenannten Pulverhefen, die Fähigkeit fehlt, sich abzulagern, auch nicht entbittert und nicht durch Verfahren der gewöhnlichen Dekantierung gewaschen werden können.

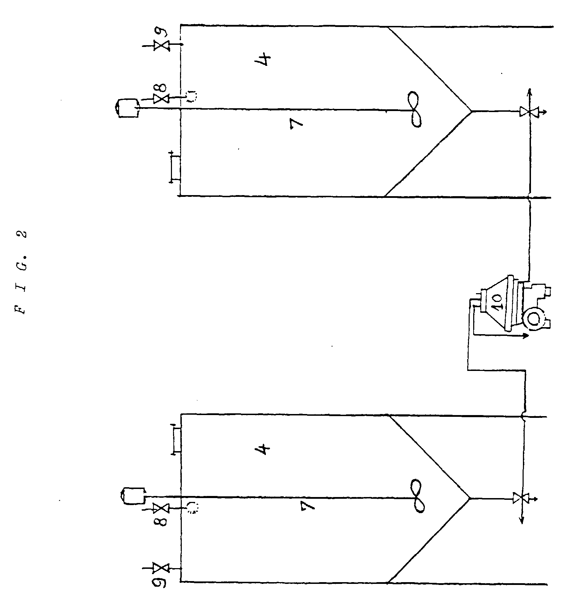

Nachstehend wird das erfindungsgemäße Verfahren zur Erzeugung von Bäckerhefe aus obergärigen Pulverhefen unter Bezug auf die Vorrichtung gemäß dem Schema der Fig. 2 erläutert.

Die Bottiche 4 zum Waschen und zur Entbitterung sind gleich wie beim schon beschriebenen ersten Verfahren. Sie weisen alle neben dem erwähnten Rührwerk 7 auch einen Wasserzufluß 8 und einen Luftzufluß 9 auf. Das Trennen der Phasen wird jedoch durch Zentrifugieren mit Hilfe des Separators und nicht durch Sedimentation ausgeführt. Auf einer Laboratoriumszentrifuge wurde bestätigt, daß die Trennung vollkommen ist.

Gesammelte obergärige Pulverhefe wird mit 0,1% Lösung Na₂CO₃ oder 0,1% Lösung NaHCO₃ im Verhältnis 1:3 bis 1:5 fünf Minuten in einem ersten Bottich 4 zur Entbitterung gemischt, dann wird mittels eines Separators 10 getrennt. Die erhaltene Hefe gibt man in einen zweiten Bottich 4 zur Entbitterung und zum Waschen, wo sie mit Wasser im Verhältnis 1:3 bis 1:5 fünf Minuten gemischt wird, und dann wird sie im Separator 10 vom Wasser getrennt und dann wieder in den ersten Bottich 4 zum Waschen und zur Entbitterung gepumpt, wo sie wieder mit Wasser gewaschen wird und so macht man dies drei bis viermal.

Die so gewaschene und entbitterte Hefe wird dann mit Hefemilch aus dem Verfahren der Erzeugung von Bäckerhefe in der Quantität bis 50% gemischt, dann wird auf einem Vakuumfilter filtriert, die erhaltene Hefe verpackt und auf den Markt geschickt.

## Patentansprüche

1. Verfahren zur industriellen Erzeugung von Bäckerhefe, bei dem Bierhefe nach Beendigung des Fermentationsprozesses durch Abtrennung vom Hopfenharz und Protein gereinigt wird, wonach die gereinigte Hefe mit einer 0,1 % Na₂CO₃- oder NaHCO₃-Lösung im Verhältnis 1:3 bis 1:5 vermischt und dann stehengelassen wird, wonach man dekantiert und die erhaltene Hefe mehrmals mit Wasser im Verhältnis 1:3 bis 1:5 vermischt, wäscht und die Hefe abdekantiert, wonach die abdekantierte Hefe in einer Menge bis zu 50 % mit Hefemilch aus Bäckerhefe vermischt und dann die Mischung filtriert wird.

2. Verfahren nach Anspruch 1 unter Verwendung von obergärigen Pulverhefen,
dadurch gekennzeichnet,
daß die Pulverhefen gleich mit der 0,1 % Na₂CO₃- oder NaHCO₃-Lösung vermischt wird, wonach die Mischung zentrifugiert und die erhaltene Hefe mehrmals gewaschen wird.

3. Verfahren nach Anspruch 1 oder 2,
dadurch gekennzeichnet,
daß das Vermischen mit der 0,1 % Na₂CO₃- oder NaHCO₃-Lösung während fünf Minuten erfolgt.

4. Verfahren nach Anspruch 1,
dadurch gekennzeichnet,
daß das Stehenlassen zehn Minuten dauert.

5. Verfahren nach einem der Ansprüche 1 bis 4,
dadurch gekennzeichnet,
daß das Waschen drei- bis fünfmal vorgenommen wird.

## Claims

1. Method for the industrial production of bakers yeast, in which after the completion of the fermentation process, brewers yeast is purified by the separation of hop resin and protein, after which the purified yeast is mixed with a 0.1 % Na₂CO₃- or NaHCO₃-solution in a ratio of 1:3 to 1:5 and then left to stand, after which decanting is carried out and the yeast obtained is mixed several times with water in a ratio of 1:3 to 1:5, washed and the yeast is decanted off, after which the decanted yeast is mixed in a quantity of up to 50 % with yeast milk from bakers yeast and then the mixture is filtered.

2. Method according to Claim 1 using top powdered yeast, characterised in that the powdered yeast is mixed in the same way with 0.1 % Na₂CO₃- or NaHCO₃-solution, whereupon the mixture is centrifuged and the yeast obtained is washed several times.

3. Method according to Claim 1 or 2, characterised in that mixing with 0.1 % Na₂CO₃- or NaHCO₃-solution takes place for five minutes.

4. Method according to Claim 1, characterised in that the mixture is left to stand for ten minutes.

5. Method according to one of Claims 1 to 4, characterised in that washing is carried out three to five times.

## Revendications

1. Procédé pour la production industrielle de levure de boulangerie, dans duquel on purifie de la levure de bière après achèvement du processus de fermentation par séparation de la résine de houblon et de protéine, apres quoi la levure purifieé est mélangée avec une solution à 0,1% de Na₂CO₃ ou de NaHCO₃ dans le rapport 1:3 à 1:5 et est alors mise au repos, après quoi l'on décante et l'on mélange à plusieurs reprises la levure obtenue avec de l'eau dans le rapport 1:3 à 1;5, l'on lave et l'on décante la levure, après quoi l'on mélange la levure décantée, dans une quantité allant jusqu'à 50%, avec du lait de levure en provenance de levure de bière et l'on filtre alors le mélange.

2. Procédé selon la revendication 1, utilisant des levures pulvérulentes surfermentées, caractérisé en ce que les levures de bière sont immédiatement mélangées avec une solution à 0,1% de Na₂CO₃ ou de NaHCO₃, après quoi le mélange est centrifugé et la levure obtenue est lavée à plusieurs reprises.

3. Procédé selon la revendication 1 ou 2, caractérisé en ce que le mélange avec la solution à 0,1% de Na₂CO₃ ou de NaHCO₃ a lieu pendant cinq minutes.

4. Procédé selon la revendication 1, caractérisé en ce que la période de mise au repos dure 10 minutes.

5. Procédé selon l'une quelconque des revendications 1 à 4, caractérisé en ce que le lavage est exécuté de trois à cinq reprises.
